# NEUE EUROPÄISCHE PATENTSCHRIFT

(11) **EP 1 075 832 B2**
(45) Veröffentlichungstag und Bekanntmachung des Hinweises auf die Entscheidung über den Einspruch: **11.08.2010**
(45) Hinweis auf die Patenterteilung: 22.08.2001
(21) Anmeldenummer: 00114815.4
(22) Anmeldetag: 11.07.2000
(51) Int. Cl.: A61K 8/81, A61Q 5/00

(54) **Volumengebendes Haarbehandlungsmittel**
Volume giving hair treating composition
Composition pour donner du volume aux cheveux

(30) Priorität: 07.08.1999 DE 19937386
(43) Veröffentlichungstag der Anmeldung: 14.02.2001
(73) Patentinhaber: Wella Aktiengesellschaft, 64274 Darmstadt (DE)
(72) Erfinder: Lede, Michael, 63225 Langen (DE); Birkel, Susanne, 95496 Glashütten (DE); Franzke, Michael, 64380 Rossdorf (DE); Henze, Hildegard, 64297 Darmstadt (DE)
(74) Vertreter: Hirsch, Uwe Thomas M.H.

(56) Entgegenhaltungen:
- EP- - 0 966 947
- EP-A- 0 074 191
- EP-A- 0 524 346
- WO- -96//19971
- WO-A-95/00104
- WO-A-96/06592
- WO-A-96/19971
- US- - 4 521 404
- US- - 5 182 098
- COSMETICS &TOILETRIES Bd. 103, Mai 1988, Seite 88
- L.R.ROBBINS CHEMICAL+PHYS. BEHAVIOR OF HUMAN HAIR 1997, Seite 360

## Beschreibung

Gegenstand der Erfindung ist ein Haarbehandlungsmittel mit einem Gehalt an einem Terpolymeren aus Vinylpyrrolidon, Vinylcaprolactam und einem basischen Acrylamidmonomer sowie einem weiteren, haarpflegenden kationaktiven Stoff mit den Merkmalen von Anspruch 1.

Haarbehandlungsmittel, die dem Haar mehr Volumen und Halt geben, sind bekannt. Die für diese Zwecke üblicherweise eingesetzten kosmetischen Polymere zeigen in wässrigen oder alkoholischen Lösungen gute Festigungseigenschaften, die nach der Anwendung mehr oder weniger gut die Haare verformen und festigen und die dem Haar zusätzlich auch mehr Volumen geben können. Häufig hält dieser Effekt aber nicht lange an und schon beim Durchkämmen der Haare geht der erwünschte Volumeneffekt wieder teilweise verloren. Insbesondere bei feinem Haar fällt die Frisur bereits innerhalb weniger Stunden wieder teilweise zusammen, d.h. der Volumeneffekt hält nicht über einen ganzen Tag von morgens bis abends an. Viele der festigenden oder Volumen gebenden Polymere haben darüberhinaus häufig unerwünschte Nebeneffekte, die sich dadurch bemerkbar machen, daß das behandelte Haar einen zu rauhen Griff, eine zu hohe Belastung oder eine ungenügende Elastizität aufweist oder sich zuviele sichtbare Rückstände auf dem Haar bilden.

Aus der WO 96/19971 sind Terpolymere aus Vinylpyrrolidon, Vinylcaprolactam und 3-(N-Dimethylaminopropyl)-methacrylamid bekannt sowie deren Verwendung in haarfestigenden Mitteln, insbesondere in Aerosol- und Pumpsprays. Diese Polymere sind besonders für einen Einsatz in wasserhaltigen Sprayrezepturen mit einem reduzierten Gehalt an leicht flüchtigen organischen Bestandteilen (low VOC-Sprays) geeignet. Die Polymere haben gute festigende Eigenschaften, verleihen dem Haar aber einen relativen rauhen Griff und eine relativ hohe Belastung.

Es bestand daher die Aufgabe, ein Haarbehandlungsmittel zur Verfügung zu stellen, welches die Vorteile eines typischen Stylingmittels aufweist, sich insbesondere durch einen langanhaltenden Volumeneffekt auszeichnet, gleichzeitig aber auch gute haarpflegende Eigenschaften, insbesondere hinsichtlich Elastizität, geringer Belastung und geringer Rückstände gewährleistet. Außerdem sollen die verwendeten Komponenten des Mittels miteinander verträglich sein, d.h. das Mittel soll möglichst homogen und klar sein und eine unbeabsichtigte Ausfällung von Rezepturbestandteilen soll vermieden werden.

Es wurde nun gefunden, daß die Aufgabe gelöst wird durch ein Haarbehandlungsmittel mit den Merkmalen von Anspruch 1.

Das Terpolymer (A) ist in dem erfindungsgemäßen Mittel in einer Menge von 0,01 bis 20, besonders bevorzugt von 0,1 bis 5 Gew.-% und das kationische Polymer (B) in einer Menge von 0,01 bis 10, besonders bevorzugt von 0,05 bis 5 Gew.-% enthalten.

Geeignete Terpolymere (A) enthalten Dimethylaminopropylmethacrylamid. Die Herstellung eines derartigen Polymers wird in der WO 96/19971 beschrieben und ist unter der Bezeichnung Aquaflex^{®} SF 40 (ISP) im Handel erhältlich.

Der kationaktive Stoff (B) ist ein Polymer, das auf Grund von kationischen Gruppen, insbesondere quaternären Amingruppen eine Substantivität zu menschlichem Haar aufweist. Geeignete kationaktive Stoffe sind kationische Polymere.

Geeignete kationaktive Tenside sind Tenside, welche eine quaternäre Ammoniumgruppe enthalten. Dabei kann es sich um kationische oder um amphotere, betainische Tenside handeln. Geeignete kationische Tenside enthalten Aminogruppen oder quaternisierte hydrophile Ammoniumgruppen, welche in Lösung eine positive Ladung tragen und durch die allgemeine Formel (I) dargestellt werden können,

W⁽⁺⁾R¹R²R³R⁴ X⁽⁻⁾ (I)

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁻ ein Anion darstellt, beispielsweise ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie beispielsweise weitere Aminogruppen enthalten.

Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt sind Cetyltrimethylammoniumchlorid, das beispielsweise in Form einer 26-prozentigen wässrigen Lösung unter der Handelsbezeichnung Dehyquart^{®} A von der Firma Henkel KGaA, Düsseldorf/Deutschland und unter der Handelsbezeichnung Genamin^{®} CTAC von der Firma Hoechst AG, Frankfurt/Deutschland sowie in Form einer 50prozentigen Lösung in Isopropanol unter der Handelsbezeichnung Arquad^{®} 16-50 von der Firma Akzo Chemicals GmbH, Düren/Deutschland vertrieben wird.

Geeignete Polymere der Komponente (B) enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quatemisiertes Vinylpyrrolidon/Dimethylaminoethylmethacrylat Copolymer (Polyquaternium-11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen LUVIQUAT^{®} HM 550 vertriebene Copolymer aus Polyvinylpyrrolidon und Imidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid, das von der Firma ISP/USA unter dem Handelsnamen Gaffix^{®} VC 713 vertriebene Terpolymer aus Vinylpyrrolidon, Dimethylaminoethylmethacrylat und Vinylcaprolactam und das von der Firma Gaf unter dem Handelsnamen Gafquat^{®} HS 100 vertriebene Vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (III)

G-O-B-N⁺R^{a}R^{b}R^{c} X⁻ (III)

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X ist ein übliches Gegenanion, hat die gleiche Bedeutung wie bei Formel (I) und ist vorzugsweise Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar R vertrieben und hat die INCI-Bezeichnung Guar Hydroxypropyltrimonium Chloride.

Bevorzugt sind solche Polymere, die eine ausreichende Alkohollöslichkeit besitzen, um in dem erfindungsgemäßen Mittel in vollständig gelöster Form vorzuliegen. Die kationische Ladungsdichte beträgt vorzugsweise 1 bis 7 meq/g.

In einer bevorzugten Ausführungsform enthält das erfindungsgemäße Mittel zusätzlich mindestens ein nichtionisches Tensid. Das nichtionische Tensid ist vorzugsweise in einer Menge von 0,01 bis 15, besonders bevorzugt von 0,05 bis 5 Gew.-% enthalten.

Bevorzugt sind dabei ethoxylierte Tenside, wobei die Anzahl der Ethylenoxid-Einheiten zwischen 1 bis 1000, bevorzugt zwischen 1 bis 300, besonders bevorzugt zwischen 1 und 15 liegt. Bevorzugt werden Fettalkoholethoxylate, Fettaminethoxylate, Fettsäurealkanolamidethoxylate und Fettsäureesterethoxylate mit jeweils 1 bis 15 Ethylenoxideinheiten. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol^{®} von der Firma Henkel oder unter der Typenbezeichnung Brij^{®} von der Firma ICI Surfactants vertrieben werden, sind für das erfindungsgemäße Haarbehandlungsmittel geeignet.

Unter den Fettsäureesterethoxylaten sind vor allem Diglyceridethoxylate zu nennen wie das von der ICI Surfactants unter dem Handelnamen Arlatone^{®} G vertriebene, mit 25 Ethylenoxid-Einheiten ethoxylierte Rizinusöl mit dem INCI-Namen PEG-25 Hydrogenated Castor Oil, das von der BASF unter dem Handelnamen Cremophor^{®} El vertriebene, mit 35 Ethylenoxid-Einheiten ethoxylierte Rizinusöl mit dem INCI-Namen PEG-35 Castor Oil, das von der BASF unter dem Handelsnamen Cremophor^{®} RH 410 vertriebene, mit 40 Ethylenoxid-Einheiten ethoxylierte, hydrierte Rizinusöl mit dem INCI-Namen PEG-40 Hydrogenated Castor Oil, und die von der Firma Witco Surfactants unter dem Namen Rewoderm^{®} LI vertriebenen Rohstoffe zu nennen.

Weiterhin können die als nicht-ionische Tenside bekannten ethoxylierten Fettsäurezuckerester, insbesondere der ethoxylierte Sorbitanfettsäureester, aber auch nicht ethoxylierte Tenside, wie die Fettsäurezuckerester, die von der Firma ICI Surfactants unter dem Handelsnamen Tween^{®} und Arlacel^{®} vertrieben werden sowie die Alkylpolyglycoside, die von der Firma Henkel unter dem Handelsnamen Plantaren^{®} oder Plantacare^{®} oder von der Firma Seppic unter dem Handelsnamen Oramix^{®} vertrieben werden, für die erfindungsgemäße kosmetische Zubereitung eingesetzt werden.

Generell läßt sich sagen, daß nicht-ionische Tenside für das erfindungsgemäße Haarbehandlungsmittel geeignet sind, wenn sie einen HLB-Wert von maximal 20, vorzugsweise von 5 bis 18 aufweisen. Besonders bevorzugt ist ein Gemisch von zwei Tensiden, wobei ein Tensid einen mittleren HLB-Wert von ca. 8 bis 11 und das andere Tensid einen höheren HLB-Wert von ca. 14 bis 17 aufweist. Geeignet ist beispielsweise eine Mischung aus Tetraethoxylaurylether (HLB 9,5) und Polysorbate-40 (HLB 15,6).

Das erfindungsgemäße Mittel enthält 0,01 bis 15 Gew.-%, vorzugsweise 0,5 bis 10 Gew.-% mindestens eines synthetischen oder natürlichen nichtionischen filmbildenden Polymers. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Unter filmbildenden Polymeren werden erfindungsgemäß solche Polymere verstanden, die bei Anwendung in 0,01 bis 5%-iger wässriger, alkoholischer oder wässrig-alkoholischer Lösung in der Lage sind, auf dem Haar einen Polymerfilm abzuscheiden.

Geeignete synthetische, nichtionische filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignet sind z.B. Homopolymere des Vinylcaprolactams, des Vinylpyrrolidons oder des N-Vinyliformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel® 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise unter den Handelsbezeichnungen Ucon^{®} der Union Carbide vertrieben werden. Besonders bevorzugt sind Polyvinylpyrrolidon und Polyvinylpyrrolidon/Vinylacetat Copolymere.

Geeignete natürliche filmbildende Polymere sind z.B. Cellulosederivate, z.B. Hydroxypropylcellulose mit einem Molekulargewicht von 30.000 bis 50.000 g/mol, welche beispielsweise unter der Handelsbezeichnung Nisso Sl^{®} von der Firma Lehmann & Voss, Hamburg, vertrieben wird.

Das erfindungsgemäße Mittel wird bevorzugt in einem wässrigen, einem alkoholischen oder in einem wässrig-alkoholischen Medium mit vorzugsweise mindestens 10 Gew.-% Wasser konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Das erfindungsgemäße Mittel kann in einem pH-Bereich von 2,0 bis 9,5 vorliegen. Besonders bevorzugt ist der pH-Bereich zwischen 2,5 und 8.

Als zusätzliche Co-Solventien können organische Lösungsmittel oder ein Gemisch aus Lösungsmitteln mit einem Siedepunkt unter 400°C in einer Menge von 0,1 bis 15 Gew.-% bevorzugt von 1 bis 10 Gew.-% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-%.

Das erfindungsgemäße Mittel kann darüber hinaus die für Haarbehandlungsmittel üblichen Zusatzbestandteile enthalten, zum Beispiel Netzmittel oder Emulgatoren aus den Klassen der anionischen oder amphoteren oberflächenaktiven Tenside, wie Fettalkoholsulfate, Alkylbenzolsulfonate, Alkyltrimethylammoniumsalze, Alkylbetaine, in einer Menge von 0,1 bis 15 Gew.-%; Feuchthaltemittel; Parfümöle in einer Menge von 0,1 bis 0,5 Gew.-%; Trübungsmittel, wie zum Beispiel Ethylenglykoldistearat, in einer Menge von etwa 0,2 bis 5,0 Gew.-%; Perlglanzmittel, wie zum Beispiel ein Gemisch aus Fettsäuremonoalkylolamid und Ethylenglykoldistearat, in einer Menge von etwa 1,0 bis 10 Gew.-%; bakterizide und fungizide Wirkstoffe wie zum Beispiel 2,4,4-Trichlor-2-hydroxydiphenylether oder Methylchlorisothiazolion, in einer Menge von 0,01 bis 1,0 Gew.-%; Verdickungsmittel, wie beispielsweise Kokosfettsäurediethanolamid, in einer Menge von etwa 0,2 bis 3,0 Gew.-%, Puffersubstanzen, wie beispielsweise Natriumcitrat oder Natriumphosphat, in einer Menge von 0,1 bis 1,0 Gew.-%; Anfärbestoffe, wie zum Beispiel Fluorescein Natriumsalz, in einer Menge von etwa 0,1 bis 1,0 Gew.-%; Pflegestoffe, wie zum Beispiel Pflanzen- und Kräuterextrakte, Protein- und Seidenhydrolysate, Lanolinderivate, in einer Menge von 0,1 bis 5 Gew.-%; physiologisch verträgliche Silikonderivate, wie zum Beispiel flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; direktziehende Haarfarbstoffe, Haarfarbstoffe, die oxidativ entwickelt werden, Oxidationsmittel, Reduktionsmittel, Fettalkohole, Glanzgeber, Vitamine, Weichmacher, Kämmbarkeitsverbesserer, rückfettende Agenzien und Entschäumer.

Das erfmdungsgemäße Mittel kann in verschiedenen Applikationsformen Anwendung finden, nämlich als Lotion oder als Non-Aerosol Sprühlotion, welches mittels einer mechanischen Vorrichtung zum Versprühen zum Einsatz kommt. Auch ein Einsatz in Form einer mit einem üblichen Verdicker verdickten Lotion ist möglich.

Wenn das erfindungsgemäße Mittel in Form einer festigenden Haarlotion vorliegt, so liegt es als nicht-viskose Lösung, Dispersion oder Emulsion mit einem Gehalt an mindestens 10 Gew.-%, vorzugsweise 20 bis 95 Gew.-% eines kosmetisch verträglichen Alkohols vor. Als Alkohole können insbesondere die für kosmetische Zwecke üblicherweise verwendeten niederen Alkohole mit 1 bis 4 Kohlenstoffatomen wie zum Beispiel Ethanol und Isopropanol verwendet werden. Die erfindungsgemäße Lotion wird in einer Portionsflasche abgepackt und kann aus dieser direkt auf das Haar aufgetragen werden. Zur Erzielung eines Volumeneffektes direkt vom Haaransatz an, kann die Verpackung mit einem Nozzel ausgerüstet sein, mittels welchem ein Auftragen direkt auf dem Haaransatz möglich ist.

Eine besonders bevorzugte Applikationsform ist eine Non-Aerosol Sprühlotion. Hierbei wird das erfindungsgemäße Mittel mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

In einer besonders bevorzugten Ausführungsform liegt das erfindungsgemäße Mittel in Form einer wässrigen, alkoholischen oder wässrig-alkoholischen Non-Aerosol Sprühlotion in Kombination mit einer geeigneten mechanischen Vorrichtung zum Versprühen oder in Form einer wässrigen, alkoholischen oder wässrig-alkoholischen Lotion vor mit einem Gehalt an
(A) 0,01 bis 20, vorzugsweise 0,1 bis 5 Gew.-%, mindestens eines Terpolymeren aus Vinylpyrrolidon, Vinylcaprolactam und Dimethylaminopropylmethacrylamid,
(B) 0,01 bis 10, vorzugsweise 0,05 bis 5 Gew.-% mindestens eines weiteren, haarpflegenden, kationischen Polymeren
(C) 0,01 bis 15, vorzugsweise 0,5 bis 10 Gew.-% mindestens eines filmbildenden, nichtionischen Polymeren und
(D) 0,01 bis 10, vorzugsweise 0,05 bis 5 Gew.-% mindestens eines kationischen Tensids,
wobei die Mengenverhältnisse der Komponenten (A) bis (D) vorzugsweise so eingestellt sind, daß eine klare Lösung resultiert.

Das erfindungsgemäße kosmetische Mittel wird angewendet, indem es in einer zur Erzielung des Volumeneffektes ausreichenden Menge auf feuchtes, handtuchtrockenes Haar aufgetragen wird. Anschließend kann die Frisur in üblicher Weise geformt werden bzw. können die Haare eingelegt und zum Schluss trocken gefönt werden. Es ist aber auch möglich, zum Auffrischen des Volumeneffektes das Mittel direkt auf trockenem Haar anzuwenden.

Das nachfolgende erfindungsgemäße Beispiel soll den Gegenstand der Erfindung näher erläutern.

### Beispiele

### Beispiel 1 (nicht erfindungsgemäß): Aerosol-Schaumfestiger

| | 1A | 1B |
|---|---|---|
| Polyquaternium-11 | - | 1,8 g |
| Vinylcaprolactam/PVP/DMAPA Copolymer | 1,8 g | - |
| Cetyltrimethylammoniumchlorid | 0,1 g | 0,1 g |
| Laureth-4 | 0,18 g | 0,18 g |
| Parfüm | 0,2 g | 0,2 g |
| Ethanol | 8,9 g | 8,9 g |
| Wasser | Ad 100 g | Ad 100 g |

Die Wirkstoffmischung wurde im Verhältnis 95:5 mit Propan/Butan 5.0 als Treibmittel in einer Aerosoldose mit Schaumventil abgefüllt.

In Halbseitenversuchen wurde jeweils eine Hälfte der Haare einer Testperson mit dem erfindungsgemäßen Aerosol-Schaum 1A und die andere Hälfte der Haare mit dem nicht erfindungsmäßen Schaum 1B behandelt. Die behandelten Haare wurden durch ausgebildete Friseurfachkräfte sensorisch beurteilt. Dabei wurden die folgenden Beurteilungen erhalten:
Vergleich 1A gegen 1B:
   Der Griff ist sowohl im feuchten als auch im trockenen Haar bei Muster 1A besser. Die Einlegbarkeit ist für Muster 1A besser und es wird für Muster 1A weniger Abstauben des Polymers vom trockenen Haar beobachtet.

### Beispiel 2 (nicht erfindungsgemäß): Pump-Sprühlotion

| | 2A | 2B |
|---|---|---|
| PVP/VA Copolymer | - | 2,5 g |
| Vinylcaprolactam/PVP/DMAPA Copolymer | 1,5 g | - |
| Cetyltrimethylammoniumchlorid | 0,2 g | 0,2 g |
| PEG-40 Hydrogenated Castor Oil | 0,2 g | 0,2 g |
| Parfüm | 0,25 g | 0,25 g |
| Ethanol | 27 g | 27 g |
| Wasser | Ad 100 g | Ad 100 g |

In Halbseitenversuchen wurde jeweils eine Hälfte der Haare einer Testperson mit der erfindungsgemäßen Sprühlotion 2A und die andere Hälfte der Haare mit der nicht erfindungsgemäßen Sprühlotion 2B behandelt. Die behandelten Haare wurden durch ausgebildete Friseurfachkräfte sensorisch beurteilt. Dabei wurden die folgenden Beurteilungen erhalten:
Vergleich 2A gegen 2B:
   Trotz reduzierter Polymermenge wurde für Muster 2A eine stärkere Festigung bepbachtet. Für Muster 2A ergab sich ein verbessertes Sprühbild.

### Beispiel 3 (nicht erfindungsgemäß): Pump-Schaumfestiger

| | 3A | 3B | 3C |
|---|---|---|---|
| Polyvinylpyrrolidon (K80) | - | - | 1,4 g |
| Vinylcaprolactam/PVP/ DMAPA Copolymer | 1,4 g | 0,9 g | - |
| Cocamidopropyl Hydroxysultaine | 0,6 g | 0,6 g | 0,6 g |
| Cetrimoniumchlorid | 0,25 g | 0,25 g | 0,25 g |
| Betain | 0,1 g | 0,1 g | 0,1 g |
| Zitronensäure | 0,1 g | 0,1 g | 0,1 g |
| Parfüm | 0,15 g | 0,15 g | 0,15 g |
| Ethanol | 2,5 g | 2,5 g | 2,5 g |
| Wasser | Ad 100 g | Ad 100 g | Ad 100 g |

In Halbseitenversuchen wurde jeweils eine Hälfte der Haare einer Testperson mit dem erfindungsgemäßen Pumpschaum 3A bzw. dem erfindungsgemäßen Pumpschaum 3B und die jeweils andere Hälfte der Haare mit dem nicht erfindungsgemäßen Pumpschaum 3C behandelt. Die behandelten Haare wurden durch ausgebildete Friseurfachkräfte sensorisch beurteilt. Dabei wurden die folgenden Beurteilungen erhalten:
Vergleich 3A gegen 3C:
   Bei vergleichbarer Polymermenge wurde für Muster 3A mehr Halt erzielt. Für Muster 3A war die Elastizität der behandelten Haare größer bei gleichzeitig weniger Belastung und einem natürlicherem Griff.
Vergleich 3B gegen 3C:
   Trotz reduzierter Polymermenge wurde für Muster 3B die gleiche Festigung erreicht. Für Muster 3B wurden weniger sichtbare Rückstände auf dem trockenen Haar beobachtet.

### Beispiel 4 (nicht erfindungsgemäß): Aerosol-Schaumfestiger

| | |
|---|---|
| Vinylcaprolactam/PVP/DMAPA Copolymer | 1,20 g |
| Polyquaternium-11 | 0,80 g |
| Betain | 0,15 g |
| PEG 25 PABA | 0,50 g |
| Cetrimoniumchlorid | 0,07 g |
| Parfüm | 0,15 g |
| Ethanol | 8,9 g |
| Wasser | Ad 100 g |

Die Wirkstoffmischung wurde im Verhältnis 95:5 mit Propan/Butan 5.0 als Treibmittel in einer Aerosoldose mit Schaumventil abgefüllt.

### Beispiel 5 (erfindungsgemäß): Pump-Sprühlotion

| | |
|---|---|
| Vinylcaprolactam/PVP/DMAPA Copolymer | 1,00 g |
| Poiyquaternium-16 | 0,20 g |
| Chitosan | 0,40 g |
| PVP/VA Copolymer | 2,50 g |
| Ameisensäure | 0,12 g |
| Cetrimoniumphosphat | 0,15 g |
| Laureth-4 | 0,10 g |
| Parfüm | 0,10 g |
| Ethanol | 33 g |
| Wasser | Ad 100 g |

Die mit der Sprühlotion behandelten Haare zeigen einen über einen ganzen Tag hinweg anhaltenden Volumeneffekt bei einer guten Festigung und einer guten Frisier- und Fönbarkeit.

### Beispiel 6 (nicht erfindungsgemäß): Pump-Schaumfestiger

| | |
|---|---|
| Vinylcaprolactam/PVP/DMAPA Copolymer | 1,1 g |
| Polyquaternium-11 | 0,4 g |
| Cocamidopropyl Hydroxysultaine | 0,6 g |
| Zitronensäure | 0,1 g |
| Betain | 0,1 g |
| Parfüm | 0,1 g |
| Ethanol | 8,9 g |
| Wasser | Ad 100 g |

## Patentansprüche

1. Haarbehandlungsmittel in Form einer wässrigen, alkoholischen oder wässrig-alkoholischen Non-Aerosol Sprühlotion in Kombination mit einer geeigneten mechanischen Vorrichtung zum Versprühen oder in Form einer wässrigen, alkoholischen oder wässrig-alkoholischen Lotion mit einem Gehalt an
(A) 0,01 bis 20 Gew.-% mindestens eines Terpolymeren aus Vinylpyrrolidon, Vinylcaprolactam und Dimethylaminopropylmethacrylamid,
(B) 0,01 bis 10 Gew.-% mindestens eines weiteren, haarpflegenden, kationischen Polymers,
(C) 0,01 bis 15 Gew.-% mindestens eines filmbildenden, nichtionischen Polymers und
(D) 0,01 bis 10 Gew.-% mindestens eines kationischen Tensids.

2. Mittel nach Anspruch 1, **dadurch gekennzeichnet, daß** das kationische Polymer ausgewählt ist aus Methylvinylimidazoliumchlorid/Vinylpyrrolidon Copolymeren, quaternisierten Vinylpyrrolidon/Dimethylaminoethylmethacrylat Copolymeren, kationisch derivatisierten Polysacchariden, Chitosan, Chitosansalzen und Chitosanderivaten.

3. Mittel nach einem der Ansprüche 1 bis 2, **dadurch gekennzeichnet, daß** das nichtionische Polymer ausgewählt ist aus Polyvinylpyrrolidon und Polyvinylpyrrolidon/Vinylacetat Copolymeren.

4. Mittel nach einem der Ansprüche 1 bis 3, **dadurch gekennzeichnet, daß** das kationische Tensid ausgewählt ist aus Verbindungen der Formel (I)
N⁽⁺⁾R¹R²R³R⁴ X⁽⁻⁾ (I)
wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁻ ein Anion darstellt.

## Claims

1. Hair-treatment composition in the form of an aqueous, alcoholic or aqueous-alcoholic non-aerosol spraying lotion in combination with a suitable mechanical device for the spraying or in the form of an aqueous, alcoholic or aqueous-alcoholic lotion with a content of
(A) 0.01 to 20% by weight of at least one terpolymer of vinylpyrrolidone, vinylcaprolactam and dimethylaminopropylmethacrylamide,
(B) 0.01 to 10% by weight of at least one further hair care cationic polymer,
(C) 0.01 to 15% by weight of at least one film-forming nonionic polymer and
(D) 0.01 to 10% by weight of at least one cationic surfactant.

2. Composition according to Claim 1, **characterized in that** the cationic polymer is chosen from methylvinylimidazolium chloride/vinylpyrrolidone copolymers, quaternized vinylpyrrolidone/dimethylaminoethyl methacrylate copolymers, cationically derivatized polysaccharides, chitosan, chitosan salts and chitosan derivatives.

3. Composition according to either of Claims 1 and 2, **characterized in that** the nonionic polymer is chosen from polyvinylpyrrolidone and polyvinylpyrrolidone/vinyl acetate copolymers.

4. Composition according to any of Claims 1 to 3, **characterized in that** the cationic surfactant is chosen from compounds of the formula (I)
N⁽⁺⁾R¹R²R³R⁴ X⁽⁻⁾ (I)
where R1 to R4, independently of one another, are aliphatic groups, aromatic groups, alkoxy groups, polyoxyalkylene groups, alkylamido groups, hydroxyalkyl groups, aryl groups or alkaryl groups having 1 to 22 carbon atoms, and X⁻ is an anion.

## Revendications

1. Composition de traitement capillaire, sous forme d'une lotion à pulvériser non aérosol, aqueuse, alcoolique ou aqueuse/alcoolique, en combinaison avec un dispositif mécanique de pulvérisation approprié, ou sous forme d'une lotion aqueuse, alcoolique ou aqueuse/alcoolique comprenant
(A) 0,01 à 20% en poids d'au moins un terpolymère de vinylpyrrolidone, de vinylcaprolactame et de diméthylaminopropylméthacrylamide,
(B) 0,01 à 10% en poids d'au moins un autre polymère cationique de soins capillaires,
(C) 0,01 à 15% en poids d'au moins un polymère filmogène non ionique et
(D) 0,01 à 10% en poids d'au moins un tensio-actif cationique.

2. Composition selon la revendication 1, **caractérisée en ce que** le polymère cationique est choisi parmi des copolymères de chlorure de méthylvinylimidazolium/vinylpyrrolidone, des copolymères quaternisés de vinylpyrrolidone/méthacrylate de diméthylaminoéthyle, des polysaccharides à dérivatisation cationique, le chitosane, des sels de chitosane et des dérivés de chitosane.

3. Composition selon l'une des revendications 1 à 2, **caractérisée en ce que** le polymère non ionique est choisi parmi la polyvinylpyrrolidone et des copolymères de polyvinylpyrrolidone/acétate de vinyle.

4. Composition selon l'une quelconque des revendications 1 à 13, **caractérisée en ce que** le tensio-actif cationique est choisi parmi des composés de formule (I)
N⁽⁺⁾R¹R²R³R⁴ X⁽⁻⁾ (I)
dans laquelle R¹ à R⁴ représentent, indépendamment les uns des autres, des groupes aliphatiques, des groupes aromatiques, des groupes alcoxy, des groupes polyoxyalkylène, des groupes alkylamido, des groupes hydroxyalkyle, des groupes aryle ou des groupes alkaryle ayant de 1 à 22 atomes de carbone, et X⁻ représente un anion.
